(19) **Europäisches Patentamt** / **European Patent Office** / **Office européen des brevets**

(11) **EP 2 752 202 B1**

(12) **EUROPEAN PATENT SPECIFICATION**

(45) Date of publication and mention of the grant of the patent:
**04.10.2017 Bulletin 2017/40**

(51) Int Cl.:
*A61K 9/48* (2006.01)     *A61K 47/10* (2017.01)
*A61K 9/107* (2006.01)     *A23L 29/10* (2016.01)
*A23L 33/115* (2016.01)     *A61K 31/23* (2006.01)
*A61K 36/05* (2006.01)     *A61K 31/202* (2006.01)

(21) Application number: **12827786.0**

(22) Date of filing: **31.08.2012**

(86) International application number:
**PCT/JP2012/072164**

(87) International publication number:
**WO 2013/031949 (07.03.2013 Gazette 2013/10)**

(54) **SOFT CAPSULE PHARMACEUTICAL PREPARATION, COMPOSITION FOR SOFT CAPSULE PHARMACEUTICAL PREPARATION, AND METHOD FOR PRODUCING SOFT CAPSULE PHARMACEUTICAL PREPARATION**

PHARMAZEUTISCHE WEICHKAPSELZUBEREITUNG, ZUSAMMENSETZUNG FÜR PHARMAZEUTISCHE WEICHKAPSELZUBEREITUNG UND VERFAHREN ZUR HERSTELLUNG EINER PHARMAZEUTISCHEN WEICHKAPSELZUBEREITUNG

PRÉPARATION PHARMACEUTIQUE POUR CAPSULES MOLLES, COMPOSITION DESTINÉE À LA PRÉPARATION PHARMACEUTIQUE POUR CAPSULES MOLLES, ET PROCÉDÉ DE PRODUCTION DE LA PRÉPARATION PHARMACEUTIQUE POUR CAPSULES MOLLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.09.2011 JP 2011191890**

(43) Date of publication of application:
**09.07.2014 Bulletin 2014/28**

(73) Proprietor: **Fujifilm Corporation**
**Minato-ku**
**Tokyo 106-8620 (JP)**

(72) Inventors:
• **HARAGUCHI, Nobuyuki**
  **Ashigarakami-gun**
  **Kanagawa 258-8577 (JP)**
• **SAKAGUCHI, Hiroyuki**
  **Ashigarakami-gun**
  **Kanagawa 258-8577 (JP)**

(74) Representative: **Hoffmann Eitle**
**Patent- und Rechtsanwälte PartmbB**
**Arabellastraße 30**
**81925 München (DE)**

(56) References cited:
**WO-A1-2010/004982     JP-A- H07 179 334**
**JP-A- H07 252 139     JP-A- H11 504 028**
**JP-A- S58 128 141     JP-A- S58 194 810**
**JP-A- S62 195 324     JP-A- 2009 114 157**
**JP-A- 2010 235 563     JP-A- 2011 012 003**
**JP-A- 2012 180 337     US-A- 4 910 021**
**US-A1- 2005 152 969     US-B1- 6 555 132**

• **DATABASE WPI Week 201109 Thomson Scientific, London, GB; AN 2011-A93493 XP002736809, -& JP 2011 012003 A (FANCL CORP) 20 January 2011 (2011-01-20)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to a soft capsule preparation, a composition for a soft capsule preparation, and a method of producing the soft capsule preparation.

Related Art

As a technology for improving the absorption of a water-insoluble physiologically-active component such as an oily component in the body, self-emulsifying compositions have been proposed. A "self-emulsifying composition" is a composition which is prepared by incorporating a physiologically active component into a composition having self-emulsifying capacity and is thereby devised to naturally undergo emulsification and dispersion only by being brought into contact with water or a digestive fluid.

For example, in Japanese Patent Application Laid-Open (JP-A) No. 2009-114157, in order to obtain a preparation in which a large amount of an oily component is blended and the use of an emulsifier is largely reduced, an emulsified composition for capsules which contains an oily component, glycerin, starch or a starch derivative and lysolecithin is disclosed. In US 6555132 B1 a gelatin-based soft capsule containing a self-emulsifying water-free composition comprising propylene glycol is disclosed. In JP-A No. 2010-235563, an emulsion composition for capsule preparations which contains an oily component, a polyhydric alcohol, a non-polyhydric alcoholic water activity-suppressing agent and an emulsifier is disclosed. Furthermore, as formulations to be applied to such a self-emulsifying composition, it is also disclosed that capsule formulations such as soft capsule are suitable from the standpoints of ease of handling and ingestibility.

SUMMARY OF INVENTION

Technical Problem

However, in those conventional soft capsule preparations that are obtained by filling a self-emulsifying composition, the storage stability is still insufficient. Specifically, since a self-emulsifying composition filled in such a soft capsule preparation is brought into a separated state in long-term storage, when the self-emulsifying composition comes into contact with water or a digestive fluid and becomes emulsified or dispersed therein, there is a problem that a size of dispersed particles of the self-emulsifying composition becomes large. In addition, since the capsule film is softened or deformed as the duration of storage is extended, there is also a problem in that an external appearance of the soft capsule is deteriorated.

The present invention was made in view of the above-described circumstances and an object of the present invention is to provide a soft capsule preparation which is obtained by encapsulation of a self-emulsifying composition and has excellent storage stability; a method of producing the soft capsule preparation; a composition for a soft capsule preparation, which is an intermediate product of the above-described soft capsule preparation; and a self-emulsifying composition and a capsule film composition that can be used in the composition for a soft capsule preparation.

Solution to Problem

Solutions to be solving the above-described problems are following.

[1] A soft capsule preparation comprising:

- a self-emulsifying composition containing an oily component, an emulsifier which is a polyglycerin fatty acid ester, 8% by mass to 20% by mass of glycerin and 2.5% by mass to 5% by mass of water; and

- a capsule film which contains gelatin and 35% by mass to 50% by mass of glycerin, in which capsule film the self-emulsifying composition is encapsulated.

[2] The soft capsule preparation according to [1], whose surface is coated with a coating agent.

[3] A method of producing the soft capsule preparation according to [1], comprising:

- preparing a self-emulsifying composition to be encapsulated into a capsule, the self-emulsifying composition containing an oily component, 20% by mass to 35% by mass of glycerin , an emulsifier which is a polyglycerin fatty acid ester and water, and a capsule film composition containing gelatin and 20% by mass to 35% by mass of glycerin;

- encapsulating the self-emulsifying composition in the capsule film composition to prepare a composition for a soft capsule preparation; and
- drying the thus obtained composition for a soft capsule preparation.

[4] The method according to [3], which comprises coating a surface of the soft capsule preparation with a coating agent after the drying.

Advantageous Effects of Invention

According to the present invention, the followings can be provided: a soft capsule preparation which is obtained by encapsulation of a self-emulsifying composition and has excellent storage stability; a method of producing the soft capsule preparation; a composition for a soft capsule preparation, which is an intermediate product of the above-described soft capsule preparation; and a self-emulsifying composition and a capsule film that are used in the composition for a soft capsule preparation.

DESCRIPTION OF EMBODIMENTS

The soft capsule preparation according to the present invention, the composition for a soft capsule preparation according to the present invention, and the method of producing the soft capsule preparation according to the present invention will now be described in detail below.
In the present invention, when reference is made to the amount of a component in a composition, in cases where the composition contains plural substances corresponding to the component, unless otherwise specified, the indicated amount means the total amount of the plural substances present in the composition.
Further, in the present description, those numerical ranges that are stated with "to" denote a range which includes the numerical values stated before and after "to" as the minimum and maximum values, respectively.
In the present description, the term "process" encompasses not only a discrete process but also a process which cannot be clearly distinguished from other processes, as long as the intended object of the process is achieved.
The soft capsule preparation according to the present invention is defined in the claims.
The composition for a soft capsule preparation according to the present invention is an intermediate product from which the soft capsule preparation according to the present invention can be suitably formed, and the soft capsule preparation according to the present invention, which is a finished product, can be obtained by drying the composition for a soft capsule preparation.
By having the above-described constitution, since separation of the encapsulated self-emulsifying composition over time as well as softening and deformation of the capsule film are effectively suppressed, the soft capsule preparation according to the present invention exhibits excellent storage stability even when it is stored for an extended period. This excellent storage stability is prominently exerted by controlling the amount of polyhydric alcohols contained in the soft capsule preparation and the amount of water contained in the self-emulsifying composition to be in the respective specific ranges prescribed in the present invention.
Further, the self-emulsifying composition and the capsule film composition that are used in the composition for a soft capsule preparation each contain a polyhydric alcohol in the specific range of amount prescribed in the present invention. When the composition for a soft capsule preparation is dried to form a soft capsule preparation, a portion of water contained in the composition for a soft capsule preparation is removed and, at the same time, a portion of the polyhydric alcohol contained in the self-emulsifying composition migrates to the capsule film side. In the soft capsule preparation, the specific amounts of polyhydric alcohol and water that are contained in the self-emulsifying composition and the specific amount of polyhydric alcohol contained in the capsule film can be attained in this manner.

(1) Soft capsule preparation

The soft capsule preparation according to the present invention is defined in the claims. Each constituent elements of the soft capsule preparation according to the present invention will be described in detail below.

<Self-emulsifying Composition>

In the soft capsule preparation according to the present invention, the self-emulsifying composition contains an emulsifier which is a polyglycerin fatty acid ester, 8% by mass to 20% by mass of glycerin an oily component, and 2.5% by mass to 5% by mass of water.

Here, the term "self-emulsifying composition" used in the present invention means a composition which has a property of naturally undergoing emulsification without requiring an external force to be applied by a mechanical operation when the composition contacts with an aqueous liquid such as water or a digestive fluid.

The indispensable components and optional components that are contained in the self-emulsifying composition of the soft capsule preparation according to the present invention will be described below.

<<Oil Component>>

The self-emulsifying composition of the soft capsule preparation contains an oily component.

The oily component is not particularly restricted and examples thereof include: a plant essential oil such as a flower oil, peppermint oil, spearmint oil or a spice oil; an oily extract derived from kola-nut, coffee, vanilla, cocoa, black tea, green tea, oolong tea or a spice; a synthetic flavor compound, a flavoring agent such as a formulated flavor composition or an arbitrary mixture thereof; an oil-soluble natural pigment such as carotenoids (e.g., lycopene and astaxanthin), a paprika pigment, an annatto dye or a chlorophyll; an ω3-unsaturated fatty acid such as docosahexaenoic acid (DHA), eicosapentaenoic acid (EPA), a fish oil containing DHA and/or EPA; linoleic acid; γ-linolenic acid; α-linolenic acid; evening primrose oil; borage oil; soybean oil; octacosanol; rosemary; sage; γ-oryzanol; β-carotene; palm carotene; perilla oil; a liposoluble vitamin such as vitamin A (retinoid), vitamin D, a tocopherol or a derivative thereof, vitamin F and vitamin K; a functional oily material such as an oil-solubilized derivative of a water-soluble vitamin; a ubiquinone including coenzyme Q10 (Co-Q10); an animal or plant oil and fat such as squalene, squalane, rapeseed oil, corn oil, olive oil, camellia oil, macadamia nut oil, mink oil, colza oil, egg-yolk oil, sesame oil, persic oil, wheat germ oil, camellia oil, flaxseed oil, cottonseed oil, perilla oil, castor oil, avocado oil, turtle oil, safflower oil, sunflower oil, rice oil, peanut oil, tea seed oil, kaya oil, rice bran oil, Chinese empress tree oil, Japanese empress tree oil, jojoba oil, germ oil, triglycerin, glycerin trioctanoate, glycerin triisopalmitate, salad oil, coconut oil, peanut oil, almond oil, hazelnut oil, walnut oil, grape seed oil, beef tallow, hardened beef tallow, hoof oil, beef bone oil, mink oil, lard, fish oil, horse tallow, mutton tallow, hardened oil, cacao butter, palm butter, hardened palm butter, palm oil, hardened palm oil, Japanese wax, Japanese wax kernel oil or hardened castor oil; a plant resin such as olibanum, rosin, copal, dammar, elemi or ester gum; an oil and fat for processed food such as a medium-chain fatty acid triglyceride having 6 to 12 carbon atoms and an arbitrary mixture of these substances.

These oily components may be used individually, or two or more thereof may be used in combination.

From the standpoints of reducing the number of capsules required for an expected effect originated from a selected oily component to be expressed (reduction in the capsule intake load) and attaining formulation stability, an amount of the oily component(s) to be contained in the self-emulsifying composition of the soft capsule preparation is preferably 50% by mass to 80% by mass, more preferably 55% by mass to 75% by mass, and still more preferably 60% by mass to 75% by mass.

<<Polyhydric Alcohol>>

The self-emulsifying composition of the soft capsule preparation contains 8% by mass to 20% by mass of a polyhydric alcohol. The polyhydric alcohol to be used in the self-emulsifying composition is glycerin.

From the standpoints of inhibiting separation of an oily component with time and suppressing an increase in dispersion particle size, an amount of the polyhydric alcohol(s) to be contained in the self-emulsifying composition of the soft capsule preparation is 8% by mass to 20% by mass, preferably 10% by mass to 20% by mass, and more preferably 14% by mass to 20% by mass.

<<Emulsifier>>

The self-emulsifying composition of the soft capsule preparation contains an emulsifier. The self-emulsifying composition may contain only one emulsifier, or two or more emulsifiers in combination.

It is preferred that the emulsifier be a water-soluble emulsifier (hydrophilic emulsifier).

Further, as the emulsifier, a water-soluble emulsifier may be used individually, or two or more water-soluble emulsifiers may be used in combination, and alternatively, a hydrophilic emulsifier and a lipophilic emulsifier may be used in combination as well.

The water-soluble emulsifier is not particularly restricted as long as it is an emulsifier which dissolves in an aqueous medium; however, the water-soluble emulsifier is preferably a non-ionic surfactant having an HLB of not less than 10, preferably not less than 12.

By using a non-ionic surfactant having the above-described HLB, the emulsification and dispersion properties of the resulting self-emulsifying composition are further improved.

The term "HLB" used herein refers to a value of hydrophilicity-hydrophobicity balance that is normally used in the field of surfactants and it can be determined by using a commonly-used formula, such as the Kawakami's equation. The Kawakami's equation is shown below.

$$HLB = 7 + 11.7\log (Mw/Mo)$$

(wherein, Mw represents the molecular weight of a hydrophilic group; and Mo represents the molecular weight of a hydrophobic group)

Alternatively, an HLB value described in a catalog or the like may also be used.

Further, as understood from the above-described equation, an emulsifier having an arbitrary HLB value can be obtained by utilizing the additive property of HLB.

The non-ionic surfactant is polyglycerin fatty acid ester. Further, the above-described emulsifier is not necessarily required to be highly purified by distillation or the like and it may also be a reaction mixture.

As a polyglycerin fatty acid ester that can be used as an emulsifier in the present invention, an ester of a polyglycerin having an average polymerization degree of not less than 2, preferably 6 to 15, more preferably 8 to 10, and a fatty acid having 8 to 18 carbon atoms, such as caprylic acid, capric acid, lauric acid, myristic acid, palmitic acid, stearic acid, oleic acid or linoleic acid, is preferred. Preferred examples of such polyglycerin fatty acid ester include hexaglycerin monooleate, hexaglycerin monostearate, hexaglycerin monopalmitate, hexaglycerin monomyristate, hexaglycerin monolaurate, decaglycerin monooleate, decaglycerin monostearate, decaglycerin monopalmitate, decaglycerin monomyristate and decaglycerin monolaurate. In the present invention, these polyglycerin fatty acid esters may be used individually or in the form of a mixture.

In cases where a polyglycerin fatty acid ester is used as an emulsifier, it is preferred that an appropriate aliphatic chain be selected with consideration of the compatibility with an oily component used in combination.

As the polyglycerin fatty acid ester, a commercial product may be employed as well. Examples thereof include NIKKOL DGMS, NIKKOL DGMO-CV, NIKKOL DGMO-90V, NIKKOL DGDO, NIKKOL DGMIS, NIKKOL DGTIS, NIKKOL Tetraglyn 1-SV, NIKKOL Tetraglyn 1-O, NIKKOL Tetraglyn 3-S, NIKKOL Tetraglyn 5-S, NIKKOL Tetraglyn 5-O, NIKKOL Hexaglyn 1-L, NIKKOL Hexaglyn 1-M, NIKKOL Hexaglyn 1-SV, NIKKOL Hexaglyn 1-O, NIKKOL Hexaglyn 3-S, NIKKOL Hexaglyn 4-B, NIKKOL Hexaglyn 5-S, NIKKOL Hexaglyn 5-O, NIKKOL Hexaglyn PR-15, NIKKOL Decaglyn 1-L, NIKKOL Decaglyn 1-M, NIKKOL Decaglyn 1-SV, NIKKOL Decaglyn 1-50SV, NIKKOL Decaglyn 1-ISV, NIKKOL Decaglyn 1-O, NIKKOL Decaglyn 1-OV, NIKKOL Decaglyn 1-LN, NIKKOL Decaglyn 2-SV, NIKKOL Decaglyn 2-ISV, NIKKOL Decaglyn 3-SV, NIKKOL Decaglyn 3-OV, NIKKOL Decaglyn 5-SV, NIKKOL Decaglyn 5-HS, NIKKOL Decaglyn 5-IS, NIKKOL Decaglyn 5-OV, NIKKOL Decaglyn 5-O-R, NIKKOL Decaglyn 7-S, NIKKOL Decaglyn 7-O, NIKKOL Decaglyn 10-SV, NIKKOL Decaglyn 10-IS, NIKKOL Decaglyn 10-OV, NIKKOL Decaglyn 10-MAC and NIKKOL Decaglyn PR-20, all of which are manufactured by Nikko Chemicals Co., Ltd.; RYOTO POLYGLYESTER L-10D, L-7D, M-10D, M-7D, P-8D, S-28D, S-24D, SWA-20D, SWA-15D, SWA-10D, O-5 O-15D, B-100D, B-70D and ER-60D, all of which are manufactured by Mitsubishi-Kagaku Foods Corporation; SUNSOFT Q-17UL, SUNSOFT Q-14S and SUNSOFT A-141C, all of which are manufactured by Taiyo Kagaku Co., Ltd.; and POEM DO-100, POEM J-0021 and POEM J-0381V, all of which are manufactured by Riken Vitamin Co., Ltd.

From the standpoint of the initial particle size of the self-emulsifying composition after being emulsified as well as the standpoint of suppressing separation of the oily component with time, an amount of the emulsifier(s) to be contained in the self-emulsifying composition of the soft capsule preparation is preferably 6% by mass to 16% by mass, and more preferably 9% by mass to 13% by mass.

The emulsifier content is set from the standpoints of the stability of the self-emulsifying composition, the particle size of the self-emulsifying composition after being emulsified and the stability of the resulting emulsion.

«Water»

The self-emulsifying composition of the soft capsule preparation contains 2.5% by mass to 5% by mass of water. This water may be any drinkable water as long as it has been appropriately treated for removal of foreign matters so as to be used in ordinary food articles.

From the standpoint of the particle size of the self-emulsifying composition after being emulsified as well as the standpoint of suppressing separation of oily component with time, the amount of the water to be contained in the self-emulsifying composition of the soft capsule preparation is 2.5% by mass to 5% by mass, and more preferably 3% by mass to 4% by mass.

«Other Components»

The self-emulsifying composition of the soft capsule preparation may further contain other component(s) as required.

Examples of the other component(s) include a flavoring agent, an antioxidant and a variety of extracts such as bilberry extract powder.

Further, in the production of a capsule preparation, from the standpoint of allowing the resulting capsule preparation immediately after its production to retain water in a preferred range even when a drying process is performed, it is also effective to further blend a component contributing to water-retaining capacity (for example, an amino acid, a saccharide or a polysaccharide) in the self-emulsifying composition.

«Physical Properties of Self-emulsifying Composition»

When brought into contact with water, the self-emulsifying composition is emulsified and dispersed to form an emulsion having a dispersion particle size at which good in vivo absorption can be exerted.

From the standpoint of allowing an oil-soluble/liposoluble physiologically active component to be efficiently absorbed to the body as an oily component, the size of the dispersed particles (volume-average particle size) at the time of self-emulsification (hereinafter, may be referred to as "re-emulsification") of the self-emulsifying composition is preferably 800 nm or smaller, and more preferably 600 nm or smaller.

The size of the dispersed particles of the self-emulsifying composition at the time of re-emulsification can be evaluated by the following method.

That is, 0.15 parts by mass of the self-emulsifying composition is brought into contact with 40 parts by mass of water, the resultant is gently stirred at 25°C for 5 minutes using a magnetic stirrer, and then the resulting dispersion is measured by a dynamic light scattering method. Examples of commercially available measuring apparatus that utilizes dynamic light scattering include a dynamic light scattering-type particle size distribution analyzer LB550 (manufactured by HORIBA Ltd.), a concentration/thickness-type particle size analyzer FPAR-1000, and NANOTRAC UPA (manufactured by Nikkiso Co., Ltd.).

In the present invention, the particle size is measured at 25°C using the concentration/thickness-type particle size analyzer FPAR-1000 and evaluated along with the monodispersity.

From the standpoint of the operability in the process of combining the self-emulsifying composition with a capsule film to produce a soft capsule preparation, the viscosity of the self-emulsifying composition is preferably 40 Pa·s or less, and more preferably 30 Pa·s or less.

The viscosity of the self-emulsifying composition is defined as a value measured by using a rheometer (Bohlin Gemini HR nano-rheometer system) at 40°C.

The viscosity of the self-emulsifying composition may be adjusted in the above-described range by any means and, for example, the viscosity of the self-emulsifying composition can be appropriately adjusted by using a thickening agent for viscosity adjustment, such as a polysaccharide, or by finely adjusting the types and amounts of the respective components contained therein.

<Capsule Film>

The capsule film of the soft capsule preparation according to the present invention contains 35% by mass to 50% by mass of a polyhydric alcohol and gelatin. In the soft capsule preparation according to the present invention, the above-described self-emulsifying composition is encapsulated in the capsule film.

The indispensable components and optional components that are contained in the capsule film of the soft capsule preparation according to the present invention will be described below.

<<Polyhydric Alcohol>>

The capsule film contains 35% by mass to 50% by mass of a polyhydric alcohol.

Examples of the polyhydric alcohol include the same ones as those mentioned above in relation to the self-emulsifying composition, and preferred embodiments thereof are also the same as described above. Among polyhydric alcohols, glycerin is most preferred because of its stability.

In the capsule film, one polyhydric alcohol may be used alone, or two or more polyhydric alcohols may be used in combination.

The polyhydric alcohol(s) contained in the self-emulsifying composition may be the same as or different from the polyhydric alcohol(s) contained in the capsule film; however, they are preferably the same. In the present invention, it is particularly preferred that all of the polyhydric alcohols contained in the self-emulsifying composition and the capsule film be glycerin.

The capsule film contains 35% by mass to 50% by mass of a polyhydric alcohol and, from the standpoints of inhibiting separation of an oily component at an early stage and over time and suppressing capsule deformation, the polyhydric alcohol content is preferably 40% by mass to 50% by mass, and more preferably 45% by mass to 50% by mass.

«Gelatin»

The capsule film contains gelatin.

The gelatin is not particularly restricted as long as it can be used in a soft capsule. From the standpoint of suppressing dent formation with time on the resulting capsule, a gelatin having a jelly strength of not less than 200 blooms can be preferably used. Further, as the gelatin, a commercial product may be employed as well, and examples thereof include IXOS Series manufactured by Nitta Gelatin Inc.

The gelatin content in the capsule film is preferably 40% by mass to 60% by mass, and more preferably 40% by mass to 50% by mass.

«Water»

It is preferred that the capsule film contain water. The details thereof are the same as those described above for the water contained in the self-emulsifying composition.

The water content in the capsule film is preferably 5% by mass to 12% by mass, more preferably 5% by mass to 10% by mass, and still more preferably 5% by mass to 8% by mass.

<<Other Components>>

The capsule film may further contain other component(s) as required.

Examples of the other component(s) include pigments, such as caramel pigment, and adhesion inhibitors such as modified starch and silicon dioxide.

The shape of the soft capsule preparation according to the present invention is not particularly restricted and it may be any of the shapes that known to be of a soft capsule preparation, such as a spherical shape, a rugby ball shape, a globular shape, a triangular shape and a teardrop shape.

The amount of the self-emulsifying composition to be encapsulated in the soft capsule preparation can be set as appropriate in accordance with the amount of components to be ingested and the number of capsules.

In the soft capsule preparation according to the present invention, it is preferred that the surface thereof be coated with a coating agent. By coating the surface of the soft capsule preparation with a coating agent, the stability of the self-emulsifying composition encapsulated in the capsule is further improved. In addition, since dent formation caused by deformation, softening and the like of the capsule film can be effectively inhibited by coating the capsule surface, the external appearance is also further improved.

Examples of the coating agent that can be used in the present invention include those coating agents that contain corn protein (zein), shellac, hydroxypropyl methylcellulose (HPMC) or the like. In the coating agent, for example, a fatty acid, a glycerin fatty acid ester, glycerin, a pigment or the like can further be incorporated as well.

As the coating agent, a commercial product may also be employed. Examples thereof include, as corn protein, "Kobayashi ZEIN DP" manufactured by Kobayashi Perfumery Co., Ltd.; as HPMC, "METOLOSE food grade" manufactured by Shin-Etsu Chemical Co., Ltd.; and "SHELLAC" manufactured by Gifu Shellac Manufacturing Co., Ltd.

The amount of the coating agent to be applied is preferably 0.5% by mass to 6% by mass, more preferably 1% by mass to 5% by mass, and particularly preferably 2% by mass to 4% by mass, with respect to the total mass of the capsule.

The soft capsule preparation according to the present invention can be suitably applied to health foods, functional foods, dietary supplements or the like.

The soft capsule preparation according to the present invention can be suitably produced by the below-described method of producing a soft capsule preparation according to the present invention.

That is, the soft capsule preparation according to the present invention can be suitably produced by the processes of: (i) preparing a self-emulsifying composition, which contains an oily component, 20% by mass to 35% by mass of a polyhydric alcohol, an emulsifier and water, and a capsule film composition which contains 20% by mass to 35% by mass of a polyhydric alcohol and gelatin; (ii) preparing a composition for a soft capsule preparation (the composition for a soft capsule preparation according to the present invention) by encapsulating the self-emulsifying composition in the capsule film composition; and (iii) drying the thus obtained composition for a soft capsule preparation.

Hereinafter, the method of producing a soft capsule preparation according to the present invention will be described in detail with inclusion of the matters that relate to the composition for a soft capsule preparation according to the present invention, which is obtained as an intermediate product in the production method.

(2) Method of Producing Soft capsule preparation, Composition for Soft capsule preparation

The method of producing a soft capsule preparation according to the present invention (hereinafter, referred to as "the production method of the present invention" as appropriate) includes the processes of: preparing a self-emulsifying composition, which contains an oily component, 20% by mass to 35% by mass of a polyhydric alcohol, an emulsifier and water, and a capsule film composition which contains 20% by mass to 35% by mass of a polyhydric alcohol and gelatin (hereinafter, also referred to as "the composition preparation process"); preparing a composition for a soft capsule preparation by encapsulating the above-described self-emulsifying composition in the above-described capsule film composition (hereinafter, also referred to as "the molding process"); and drying the thus obtained composition for a soft capsule preparation (hereinafter, also referred to as "the drying process").

The composition for a soft capsule preparation obtained in the above-described molding process is the composition for a soft capsule preparation according to the present invention.

The composition for a soft capsule preparation according to the present invention is an intermediate product from which the soft capsule preparation according to the present invention can be formed, and the soft capsule preparation according to the present invention, which is a finished product, can be obtained by drying the composition for a soft capsule preparation in the drying process after the molding process.

The constituents of the production method of the present invention will each be described in detail below.

<Composition Preparation Process>

In the composition preparation process, a self-emulsifying composition which contains an oily component, 20% by mass to 35% by mass of a polyhydric alcohol, an emulsifier and water, and a capsule film composition which contains 20% by mass to 35% by mass of a polyhydric alcohol and gelatin are prepared.

<<Self-emulsifying Composition>>

As the self-emulsifying composition used in the composition for a soft capsule preparation, the self-emulsifying composition according to the present invention which contains an oily component, 20% by mass to 35% by mass of a polyhydric alcohol, an emulsifier and water is suitably applied.

With regard to the matters other than those pertaining to the contents of the polyhydric alcohol, emulsifier and water, such as the types and preferred embodiments of the indispensable components and optional components that are contained in the self-emulsifying composition of the composition for a soft capsule preparation, those matters that are described above in relation to the self-emulsifying composition of the soft capsule preparation are applied in the same manner.

In the composition for a soft capsule preparation, the amount of the oily component to be contained in the self-emulsifying composition is preferably 55% by mass to 78% by mass, and more preferably 60% by mass to 70% by mass.

In the composition for a soft capsule preparation, from the standpoint of the stability with time of the contents encapsulated in the capsule film, the amount of the polyhydric alcohol to be contained in the self-emulsifying composition is 20% by mass to 35% by mass, and more preferably 23% by mass to 32% by mass.

In the composition for a soft capsule preparation, from the standpoints of the dispersion particle size and the emulsion stability, the amount of the emulsifier to be contained in the self-emulsifying composition is preferably 5% by mass to 13% by mass, and more preferably 8% by mass to 11% by mass.

In the composition for a soft capsule preparation, the water content in the self-emulsifying composition is set such that the viscosity of the composition is reduced to a level at which the composition can be filled into the capsule film and that the water content in the self-emulsifying composition of the soft capsule preparation obtained by drying the composition for a soft capsule preparation becomes 2.5% by mass to 5% by mass. The water content is preferably 4% by mass to 10% by mass, and more preferably 4% by mass to 6% by mass. From the standpoints of reducing the load in the drying of the capsule and suppressing migration of glycerin into the capsule film at the time of drying, it is preferred that the water content in the self-emulsifying composition be set to the minimum required level.

The self-emulsifying composition of the composition for a soft capsule preparation can be prepared by, for example, a method in which a solution (oil phase) containing an oily component and an optional component(s) is slowly added to a mixed solution (aqueous phase) prepared by dissolving a polyhydric alcohol, water and an optional component(s).

<<Capsule Film Composition>>

As the capsule film composition contained in the composition for a soft capsule preparation, the capsule film composition according to the present invention which contains 20% by mass to 35% by mass of a polyhydric alcohol and gelatin is suitably employed. That is, the composition for a soft capsule preparation according to the present invention is obtained by encapsulating the self-emulsifying composition according to the present invention, which contains an oily component, 20% by mass to 35% by mass of a polyhydric alcohol, an emulsifier and water, into the capsule film composition according to the present invention which assumes the form of a film.

It is noted here that the capsule film composition according to the present invention encompasses the form of being molded into a film as well as the form of not being molded into a specific shape.

With regard to the matters other than those pertaining to the content of the polyhydric alcohol, such as the types and preferred embodiments of the indispensable components and optional components that are contained in the capsule film composition of the composition for a soft capsule preparation, those matters that are described above in relation to the capsule film of the soft capsule preparation are applied in the same manner.

The amount of the polyhydric alcohol to be contained in the capsule film composition is 20% by mass to 35% by mass, and more preferably 22% by mass to 31% by mass.

Here, in the composition for a soft capsule preparation, the polyhydric alcohol contained in the self-emulsifying composition may be the same as or different from the polyhydric alcohol contained in the capsule film composition; however, they are preferably the same. In the present invention, it is particularly preferred that the polyhydric alcohols contained in the self-emulsifying composition and capsule film composition of the composition for a soft capsule preparation be all glycerin.

The amount of gelatin to be contained in the capsule film composition is preferably 35% by mass to 45% by mass, and more preferably 38% by mass to 43% by mass.

The capsule film composition can be prepared in the form of a solution in which the prescribed components to be contained therein are dissolved.

<Molding Process>

In the molding process, the self-emulsifying composition of the above-described composition for a soft capsule preparation is encapsulated into the above-described capsule film composition to obtain a composition for a soft capsule preparation (the composition for a soft capsule preparation according to the present invention).

In the molding process, the encapsulation of the self-emulsifying composition into the capsule film composition can be carried out by, for example, using a variety of known methods such as a rotary system, a seamless system or a plate system.

With regard to a method in which a rotary die-type automatic soft capsule production machine, which is one example of the rotary system, is used, for example, those matters disclosed in the paragraphs [0024] to [0031] of JP-ANo. 2004-351007 are applicable to this molding process in the same manner.

For the encapsulation of the self-emulsifying composition into the capsule film composition performed in the molding process, for example, a plate method in which a laminate is formed by inserting the self-emulsifying composition between two molded sheets of the capsule film composition and the thus obtained laminate is then compressed and punched out from both sides using a die can be employed as required.

<Drying Process>

In the drying process, the composition for a soft capsule preparation obtained in the above-described molding process is dried. By going through this process, a soft capsule preparation can be obtained as a finished product.

The drying method is not particularly restricted and the drying can be performed by using a known dryer such as a tumbler dryer (rotary drum-type dryer). Further, after tumbler drying, it is preferred that the resulting capsule be placed in an environment having an appropriate temperature and humidity and further dried for an appropriate duration.

The drying temperature is preferably about 25°C to 30°C and the drying humidity is preferably about 30% RH to 50% RH. The drying is preferably performed for about 3 days to 10 days.

<Coating Process>

In the production method of the present invention, by further performing a coating process after the drying process, the surface of the thus obtained soft capsule preparation may be coated with a coating agent.

This coating can be carried out by applying a coating agent to the surface of the soft capsule preparation in accordance with a conventional method using a spray coating apparatus or the like.

With regard to the coating agent and the amount thereof to be applied, those matters that are described above in relation to the soft capsule preparation of the present invention are applied in the same manner.

EXAMPLES

The present invention will now be described in detail by way of examples thereof; however, the present invention is not restricted thereto by any means. It is noted here that, unless otherwise specified, "%" is based on mass.

[Examples 1 to 16, Comparative Examples 1 to 7]

(1) Production of Soft capsule preparation

<Preparation of Self-emulsifying Composition>

A self-emulsifying composition (before drying) was prepared as follows by using the respective components shown in Table 1 or 2 in the amounts shown in Table 1 or 2.

<<Preparation of Aqueous Phase>>

An aqueous phase was prepared by dissolving an emulsifier shown in Table 1 or 2 in glycerin at 70°C, cooling the resultant to 25°C, and then adding water to obtain a uniform liquid.

<<Preparation of Oil Phase>>

An oil phase was prepared as a liquid containing only an oil shown in Table 1 or 2 and, when *Haematococcus* algae extract was used, an oil phase was prepared as a mixture of the oil shown in Table 1 or 2 and the *Haematococcus* algae extract. This mixture of oil and *Haematococcus* algae extract was prepared by dissolving the *Haematococcus* algae extract in the oil at 70°C and then cooling the resultant to 25°C.

<<Preparation of Self-emulsifying Composition>>

While stirring the thus obtained aqueous phase using an azihomomixer, the entire amount of the thus obtained oil phase was added thereto in small amounts at a time using a tube pump, thereby preparing a composition. Thereafter, the thus obtained composition was vacuum-degassed to obtain a self-emulsifying composition.

<Preparation of Capsule Film Composition>

A capsule film composition (before drying) was prepared as follows by using the respective components shown in Table 1 or 2 in the amounts shown in Table 1 or 2.
After adding gelatin to water, the resultant was allowed to swell and then heated to 70°C so as to dissolve the gelatin, thereby obtaining a solution. Then, glycerin was added to the thus obtained solution and the resulting mixture was stirred and degassed while it was warm, thereby preparing a capsule film composition.

<Production of Soft capsule preparation>

By a conventional method using a rotary die system, the above-described self-emulsifying composition (before drying) was encapsulated into the thus obtained capsule film composition (before drying) to prepare a composition for a soft capsule preparation. Then, by drying this composition for a soft capsule preparation using a tumbler dryer, a soft capsule preparation was obtained.

<<Coating>>

For the soft capsule preparations of Examples 10 to 12 and 14 to 16, the obtained capsule preparations after drying were coated with the respective coating agents shown in Table 1 using a Dria coater. The amount of the coating film was set to be 2% with respect to the mass of each capsule.
The constitutions of the self-emulsifying compositions (after drying) and the capsule films (after drying) in the respective soft capsule preparations (finished products) were as shown in Tables 1 and 2.
Further, the capsule films of the respective soft capsule preparations had a thickness in the range of 0.78 $\mu$m

to 0.82 μm.

The details of the respective components shown in Tables 1 and 2 were as follows.

<Oil Component>

Oil A: COCONARD RK (trade name), manufactured by Kao Corporation; medium-chain fatty acid triglyceride
Oil B: DHA 70G (trade name), manufactured by NISSUI; refined fish oil containing a large amount of DHA
*Haematococcus* algae extract: ASTOTS-S (trade name), manufactured by Takeda Shiki Co., Ltd.; containing 20% astaxanthin

<Polyhydric Alcohol>

Glycerin (food grade glycerin, manufactured by Kao Corporation)

<Emulsifier>

Emulsifier A: NIKKOL Decaglyn 1-L (trade name), manufactured by Nikko Chemicals Co., Ltd.; decaglycerin fatty acid ester whose aliphatic chain is lauric acid (C18 unsaturated fatty acid)
Emulsifier B: POEM J-0381V (trade name), manufactured by Riken Vitamin Co., Ltd.; decaglycerin fatty acid ester whose aliphatic chain is oleic acid (C18 unsaturated fatty acid)

<Other Component>

Gelatin (trade name: IXOS manufactured by Nitta Gelatin Inc.)

<Coating Agent>

Corn protein (trade name: Kobayashi ZEIN DP, manufactured by Kobayashi Perfumery Co., Ltd.)
HPMC (trade name: METOLOSE food grade, manufactured by Shin-Etsu Chemical Co., Ltd.; hydroxypropyl methylcellulose)
SHELLAC (manufactured by Gifu Shellac Manufacturing Co., Ltd.)

(2) Evaluation of Soft capsule preparation

Using the soft capsule preparations that were obtained in Examples 1 to 16 and Comparative Examples 1 to 7, the initial dispersibility and the storage stabilities (stability with time, dent formation with time and softening with time) were evaluated.

1. Initial Dispersibility

The initial dispersibility was evaluated by the following method based on the evaluation criteria described below.

<Evaluation Method>

A capsule was opened and its content was squeezed out into a disposable cup. Then, 100 ml of water was added thereto, and the resultant was stirred to allow the content to be dispersed, thereby obtaining a dispersion for evaluation.
The thus obtained dispersion for evaluation was visually observed and evaluated based on the following criteria. The results thereof are shown in Tables 1 and 2.

<Evaluation Criteria>

A: No separation of the oily component was observed, and the oily component was uniformly dispersed. The dispersion was highly transparent.
B: A slight separation of the oily component was observed; however, the oily component was uniformly dispersed.
C: Separation of the oily component was observed. The dispersion had a low transparency.
D: The oily component was notably separated. The dispersion was highly turbid.
E: The oily component was mostly separated. Only a small portion of the oily component was dispersed.

2. Storage Stability

2-1. Stability with Time

The stability with time was evaluated by the following method based on the evaluation criteria described below.

<Evaluation Method>

After storing each capsule in a 40°C environment for one month, a dispersion for evaluation was prepared in the same manner as in the above-described evaluation of the initial dispersibility.
The thus obtained dispersion for evaluation was visually observed, and the stability with time was evaluated based on the following criteria. The results thereof are shown in Tables 1 and 2.

<Evaluation Criteria>

A: Hardly any separation of the oily component was observed, and the oily component was uniformly dispersed. The dispersion was highly transparent.
B: Separation of the oily component was observed to some extent; however, the oily component was uniformly dispersed.
C: A relatively large amount of the oily component was observed to be separated; however, the oily component was uniformly dispersed. The dispersion had a low transparency.
D: The oily component was notably separated. The dispersion was highly turbid.
E: The oily component was mostly separated. Only a small portion of the oily component was dispersed.

2-2. Dent Formation and Softening with Time

After storing each soft capsule preparation in a 40°C environment for one month, dent formation and softening with time were evaluated based on the following criteria. The results thereof are shown in Tables 1 and 2.

<Evaluation Criteria>

A: No dent formation was observed, and the capsules did not change with time. The capsules were not deformed unless a certain amount of force was applied.
B: Of all the capsules that were evaluated, capsules having a minor dent accounted for 30% or less in number.
C: Of all the capsules that were evaluated, capsules having an obvious dent accounted for 30% or less in number.
D: Of all the capsules that were evaluated, capsules having an obvious dent accounted for 60% or less in number. The capsules were soft and deformed by a small pressure.
E: All of the evaluated capsules had a dent. The capsules were extremely soft.

12

[Table 1]

| | | | Example 1 | Example 2 | Example 3 | Example 4 | Example 5 | Example 6 | Example 7 | Example 8 | Example 9 | Example 10 | Example 11 | Example 12 | Example 13 | Example 14 | Example 15 | Example 16 |
|---|---|---|---|---|---|---|---|---|---|---|---|---|---|---|---|---|---|---|
| Self-emulsifying composition | Before drying | Oil A (%) | - | - | - | - | - | - | - | - | - | - | - | - | 60.3 | 60.3 | 60.3 | 60.3 |
| | | Oil B (%) | 63.1 | 60.3 | 55.6 | 60.3 | 60.9 | 60.1 | 59.6 | 60.3 | 60.3 | 60.3 | 60.3 | 60.3 | - | - | - | - |
| | | Haematococcus algae extract (%) | - | - | - | - | - | - | - | - | - | - | - | - | 4.3 | 4.3 | 4.3 | 4.3 |
| | | Emulsifier A (%) | - | - | - | - | - | - | - | - | - | - | - | - | 8.6 | 8.6 | 8.6 | 8.6 |
| | | Emulsifier B (%) | 9 | 8.6 | 7.9 | 8.6 | 8.7 | 8.6 | 8.5 | 8.6 | 8.6 | 8.6 | 8.6 | 8.6 | - | - | - | - |
| | | Glycerin (%) | 22.5 | 25.9 | 31.7 | 25.9 | 26.1 | 25.8 | 25.5 | 25.9 | 25.9 | 25.9 | 25.9 | 25.9 | 25.9 | 25.9 | 25.9 | 25.9 |
| | | Water (%) | 5.4 | 5.2 | 4.8 | 5.2 | 4.3 | 5.6 | 5.5 | 5.2 | 5.2 | 5.2 | 5.2 | 5.2 | 5.2 | 5.2 | 5.2 | 5.2 |
| | After drying | Oil A (%) | - | - | - | - | - | - | - | - | - | - | - | - | 70 | 70 | 70 | 70 |
| | | Oil B (%) | 79 | 72.5 | 66 | 76.3 | 73.6 | 72.1 | 71.4 | 71.8 | 71.1 | 72.5 | 72.5 | 72.5 | - | - | - | - |
| | | Haematococcus algae extract (%) | - | - | - | - | - | - | - | - | - | - | - | - | 5 | 5 | 5 | 5 |
| | | Emulsifier A (%) | - | - | - | - | - | - | - | - | - | - | - | - | 8 | 8 | 8 | 8 |
| | | Emulsifier B (%) | 11 | 10 | 9.4 | 10.9 | 10.5 | 10.3 | 10.2 | 10.3 | 9.8 | 10 | 10 | 10 | - | - | - | - |
| | | Glycerin (%) | 8 | 14 | 20 | 9 | 14.2 | 13.9 | 13.8 | 15 | 16 | 14 | 14 | 14 | 14 | 14 | 14 | 14 |
| | | Water (%) | 3 | 3.5 | 4 | 3.5 | 2.5 | 4 | 5 | 3.5 | 3.5 | 3.5 | 3.5 | 3.5 | 3 | 3 | 3 | 3 |
| Capsule film composition | Before drying | Gelatin (%) | 40 | 40 | 40 | 43.5 | 40 | 40 | 40 | 38.5 | 38 | 40 | 40 | 40 | 40 | 40 | 40 | 40 |
| | | Glycerin (%) | 28 | 28 | 28 | 22 | 28 | 28 | 28 | 30.8 | 32 | 28 | 28 | 28 | 28 | 28 | 28 | 28 |
| | | Water (%) | 32 | 32 | 32 | 34.8 | 32 | 32 | 32 | 30.8 | 30 | 32 | 32 | 32 | 32 | 32 | 32 | 32 |
| Capsule film | After drying | Gelatin (%) | 43 | 44 | 42 | 45 | 45 | 44 | 44 | 42 | 41 | 44 | 44 | 44 | 44 | 44 | 44 | 44 |
| | | Glycerin (%) | 50 | 49 | 50 | 48 | 48 | 49 | 49 | 50 | 50 | 49 | 49 | 49 | 49 | 49 | 49 | 49 |
| | | Water (%) | 7 | 7 | 8 | 7 | 7 | 7 | 7 | 8 | 8 | 7 | 7 | 7 | 7 | 7 | 7 | 7 |
| Presence/absence of coating | | | - | - | - | - | - | - | - | - | - | corn protein | HPMC | shellac | - | corn protein | HPMC | shellac |
| Evaluation | | Initial dispersibility | B | A | A | B | B | A | A | A | A | A | A | A | B | A | A | A |
| | | Stability with time | C | B | A | B | B | A | A | A | A | A | A | A | B | A | A | A |
| | | Dent formation and softening with time | C | C | C | C | C | C | C | C | C | A | A | A | C | A | A | A |

[Table 2]

| | | | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|---|
| Self-emulsifying composition | Before drying | Oil A (%) | - | - | - | - | - | - | - |
| | | Oil B (%) | 66 | 51.5 | 60.3 | 61.4 | 61.1 | 59.3 | 60.3 |
| | | Haematococcus algae extract (%) | - | - | - | - | - | - | - |
| | | Emulsifier A (%) | - | - | - | - | - | - | - |
| | | Emulsifier B (%) | 9.4 | 7.4 | 8.6 | 8.8 | 8.7 | 8.5 | 8.6 |
| | | Glycerin (%) | 18.9 | 36.8 | 25.9 | 26.3 | 26.2 | 25.4 | 25.9 |
| | | Water (%) | 5.7 | 4.4 | 5.2 | 3.5 | 3.9 | 6.8 | 5.2 |
| | After drying | Oil A (%) | - | - | - | - | - | - | 5.2 |
| | | Oil B (%) | 80.5 | 64 | 78 | 74.6 | 74 | 70.6 | 69 |
| | | Haematococcus algae extract (%) | - | - | - | - | - | - | - |
| | | Emulsifier A (%) | - | - | - | - | - | - | - |
| | | Emulsifier B (%) | 10 | 10 | 10.8 | 10.3 | 10.2 | 9.7 | 9.5 |
| | | Glycerin (%) | 6 | 22 | 7 | 14.4 | 14.3 | 13.6 | 19 |
| | | Water (%) | 3.5 | 4 | 3.5 | 1.2 | 2 | 6 | 3.5 |
| Capsule film composition | Before drying | Gelatin (%) | 40 | 40 | 46.5 | 40 | 40 | 40 | 35.7 |
| | | Glycerin (%) | 28 | 28 | 16.3 | 28 | 28 | 28 | 35.7 |
| | | Water (%) | 32 | 32 | 37.2 | 32 | 32 | 32 | 28.6 |
| Capsule film | After drying | Gelatin (%) | 55 | 45 | 47 | 44 | 44 | 42 | 39 |
| | | Glycerin (%) | 48 | 47 | 46 | 49 | 49 | 47 | 51 |
| | | Water (%) | 7 | 8 | 7 | 7 | 7 | 11 | 10 |
| Presence/absence of coating | | | - | - | - | - | - | - | - |
| Evaluation | | Initial dispersibility | E | A | B | B | B | B | A |
| | | Stability with time | E | A | D | E | D | A | A |
| | | Dent formation and softening with time | D | D | B | C | C | D | E |

As shown in Tables 1 and 2, it is understood that the soft capsule preparations of the Examples had better initial dispersibility and superior storage stability as compared to those of the Comparative Examples.

Further, as shown in the evaluation results of the soft capsule preparations of Examples 10 to 12 and 14 to 16, it is understood that the coating of the capsule surface with a coating agent exerted excellent effect in both the initial dispersion and the storage stability.

## Claims

1.   A soft capsule preparation comprising:

a self-emulsifying composition containing an oily component, an emulsifier which is a polyglycerin fatty acid ester , 8% by mass to 20% by mass of glycerin and 2.5% by mass to 5% by mass of water; and
a capsule film which contains gelatin and 35% by mass to 50% by mass of glycerin, in which capsule film the self-emulsifying composition is encapsulated.

2.   The soft capsule preparation according to claim 1 , whose surface is coated with a coating agent.

3.   A method of producing the soft capsule preparation according to claim 1, comprising:

preparing a self-emulsifying composition to be encapsulated into a capsule, the self-emulsifying composition containing an oily component, 20% by mass to 35% by mass of glycerin , an emulsifier which is a polyglycerin fatty acid ester and water, and a capsule film composition containing gelatin and 20% by mass to 35% by mass of glycerin;

encapsulating the self-emulsifying composition in the capsule film composition to prepare a composition for a soft capsule preparation; and

drying the thus obtained composition for a soft capsule preparation.

4. The method according to claim3, which comprises coating a surface of the soft capsule preparation with a coating agent after the drying.

**Patentansprüche**

1. Weichkapselzubereitung, umfassend:

eine selbstemulgierende Zusammensetzung, die eine ölige Komponente, einen Emulgator, welcher ein Polyglycerin-Fettsäureester ist, 8 Massen% bis zu 20 Massen% an Glycerin und 2,5 Massen% bis 5 Massen% an Wasser enthält; und

einen Kapselfilm, welcher Gelatine und 35 Massen% bis 50 Massen% an Glycerin enthält, wobei die selbstemulgierende Zusammensetzung in dem Kapselfilm eingekapselt ist.

2. Weichkapselzubereitung gemäß Anspruch 1, deren Oberfläche mit einem Beschichtungsmittel beschichtet ist.

3. Verfahren zum Herstellen der Weichkapselzubereitung gemäß Anspruch 1, umfassend:

Zubereiten einer selbstemulgierenden Zusammensetzung, die in einer Kapsel eingekapselt werden soll, wobei die selbstemulgierende Zusammensetzung eine ölige Komponente, 20 Massen% bis 35 Massen% an Glycerin, einen Emulgator, welcher ein Polyglycerin-Fettsäureester ist, und Wasser enthält, und eine Kapselfilmzusammensetzung, die Gelatine und 20 Massen% bis 35 Massen% an Glycerin enthält;

Einkapseln der selbstemulgierenden Zusammensetzung in der Kapselfilmzusammensetzung, so dass eine Zusammensetzung für eine Weichkapselzubereitung hergestellt wird; und

Trocknen der dadurch erhaltenen Zusammensetzung für eine Weichkapselzubereitung.

4. Verfahren gemäß Anspruch 3, das das Beschichten einer Oberfläche der Weichkapselzubereitung mit einem Beschichtungsmittel nach dem Trocknen umfasst.

**Revendications**

1. Préparation de capsule molle comprenant :

une composition auto-émulsionnante contenant un composant huileux, un émulsifiant qui est un ester d'acide gras de polyglycérine, 8 % en masse à 20 % en masse de glycérine et 2,5 % en masse à 5 % en masse d'eau ; et un film de capsule qui contient de la gélatine et 35 % en masse à 50 % en masse de glycérine, dans lequel film de capsule encapsule la composition auto-émulsionnante.

2. Préparation de capsule molle selon la revendication 1, dont la surface est revêtue d'un agent de revêtement.

3. Procédé de production de la préparation de capsule molle selon la revendication 1, comprenant :

préparer une composition auto-émulsionnante à encapsuler dans une capsule, la composition auto-émulsionnante contenant un composant huileux, 20 % en masse à 35 % en masse de glycérine, un émulsifiant qui est un ester d'acide gras de polyglycérine et de l'eau, et une composition de film de capsule contenant de la gélatine et 20 % en masse à 35 % en masse de glycérine ;

encapsuler la composition auto-émulsionnante dans la composition de film de capsule pour préparer une composition pour une préparation de capsule molle ; et

sécher la composition ainsi obtenue pour une préparation de capsule molle.

4. Procédé selon la revendication 3, qui comprend le revêtement d'une surface de la préparation de capsule molle avec un agent de revêtement après le séchage.

**EP 2 752 202 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009114157 A
- US 6555132 B1
- JP 2010235563 A
- JP 2004351007 A